Europäisches Patentamt

European Patent Office

Office européen des brevets

(11) Publication number: **0 093 116**
**B1**

⑱

(12) **EUROPEAN PATENT SPECIFICATION**

(45) Date of publication of patent specification: **09.10.85**

(21) Application number: **82901740.9**

(22) Date of filing: **28.05.82**

(86) International application number:
**PCT/SE82/00193**

(87) International publication number:
**WO 82/04264 09.12.82 Gazette 82/29**

(51) Int. Cl.⁴: **C 12 Q 1/00**

(54) **METHOD OF QUANTITATIVELY ASSAYING MICROORGANISMS OR SUBSTANCES AFFECTING THE GROWTH THEREOF.**

(30) Priority: **04.06.81 SE 8103520**

(43) Date of publication of application:
**09.11.83 Bulletin 83/45**

(45) Publication of the grant of the patent:
**09.10.85 Bulletin 85/41**

(84) Designated Contracting States:
**DE FR GB NL**

(56) References cited:
**WO-A-80/02849**
**DE-A-2 826 416**
**US-A-3 403 081**
**US-A-3 843 324**
**US-A-4 072 576**

**Methods in Enzymol., vol. 34, pp. 220-23, in particular p 224, 2nd paragraph, publ. 1974, (Ed. JAKOBY W B, WILCHEK M)**
**Proc. Nat. Acad. Sci. U.S.A., vol. 68, pp 2153-7, publ. 1971 (EDELMAN G M et al), "Cell fractionation and arrangement on fibers, beads and surfaces"**

(73) Proprietor: **ALFA-LAVAL AB**
**Box 500**
**S-147 00 Tumba (SE)**

(72) Inventor: **MATTIASSON, Bo Gustav**
**Trädgårdsmästaren 26**
**S-222 48 Lund (SE)**

(74) Representative: **Austin, Hedley William et al**
**A.A. THORNTON & CO. Northumberland House**
**303-306 High Holborn**
**London WC1V 7LE (GB)**

(56) References cited:
**Chem. & Eng. News pp 29-35, publ. 27 January 1975 (RECHNITZ G A) "Membrane bioprobe electrodes"**
**Biomed. Eng., vol. 9, pp 18-20, publ. January 1974 (UR A, BROWN D F J), "Rapid detection of bacterial activity using impedance measurements"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention is concerned with a method of quantitatively assaying a microorganism or unicellular organism present in a liquid medium, or of quantitatively assaying a substance which is capable of affecting the metabolism, positively or negatively, or a microorganism or unicellular organism.

Several procedures are available for determining the amount of a particular microorganism, but a common drawback with all these methods is that they require a long time, often several days to make one determination. A method commonly employed involves spreading a layer of substrate on a plate or glass surface and then adding the microorganism sample. At first nothing is seen in the medium, but gradually the microorganisms divide and eventually form a number of colonies which can be seen with the naked eye. Based on the assumption that one cell gives rise to one colony, the number of microorganisms originally present can be determined.

Another method to determine the amount of a particular microorganism is to use specific adsorbents containing antibodies. Such adsorbents adsorb a particular type of microorganism towards which the antibodies are specific. Microorganisms bound in this way can then be incubated in a growth medium; when they have divided sufficiently to give large groups of cells that can be seen with the naked eye, the original number of microorganisms can be determined by colony counting. The choice of the substrate to which the adsorbent and bound microorganisms are added depends on the type of microorganism that has been adsorbed, since particular microorganisms are able to grow only on certain substrates.

The present invention affords a method of quantitatively determining microorganisms within a short period of time, in many cases within two hours.

According to one aspect of the present invention, there is provided a method of quantitatively assaying a microorganism or unicellular organism present in a liquid medium, which comprises contacting the medium with an adsorbent in the form of a carrier provided with lectins and/or antibodies, which lictins and/or antibodies bind specifically to the microorganism or unicellular organism to be assayed, separating the adsorbent and the bound organism from any non-bound organisms, contacting the adsorbent and the bound organism with a substrate for the metabolism of the bound organism, measuring a chemical or physical change in the substrate which results from the metabolism of the bound organism, and assaying the amount of the bound organism from such measurement.

According to another aspect of the invention there is provided a method of quantitatively assaying a substance, in which a known amount of a microorganism or unicellular organism, the metabolism of which is affected, positively or negatively, by the substance to be assayed, is bound on an adsorbent as used according to the invention, the adsorbent and the bound organism are contacted with a liquid medium containing the substance to be assayed, a chemical or physical change in the medium resulting from the metabolism of the bound organism is measured, and the amount of the substance present in the medium is assayed from such measurement.

In carrying out the first aspect of the invention, the adsorbent is usually provided with a specific antibody to bind a particular microorganism. However, if it is desired to adsorb all the microorganisms present in a mixture of microorganisms then either a mixture of antibodies or other binding structures of broader specificity, such as lectins, are used.

By measuring the metabolic activity of known amounts of microorganisms, a calibration curve can be prepared by which the amount of microorganisms in an unknown sample may be determined.

In carrying out the second aspect of the invention, positive effects are obtained when assaying substances such as vitamins, and negative effects are given by inhibitors of metabolic activity, such as antibiotics.

By measuring the metabolic activity of a known amount of microorganisms in the presence of various concentrations of the substance being assayed, calibration curves can be prepared by which the amount of the substance in an unknown sample may be determined.

One of the chemical and physical changes by which metabolic activity may be monitored is consumption of oxygen. The amount of oxygen consumed can be measured with available analytical instruments.

Another possible variable is the pH of the substrate. The pH can be measured using a pH-meter or a pH-indicator and photometric equipment.

Another parameter is reduction equivalents formed during metabolism. These reduction equivalents are uncoupled from ordinary metabolism by, for example, artificial electron acceptors; the degree of reduction can be determined by available techniques.

Another possible parameter is the amount of carbon dioxide generated during metabolism; this amount can be measured with available analytical instruments.

For certain types of microorganisms, specific metabolites may be excreted, the finer concentration of which in the substrate can be used to determine the number of microorganisms bound to the adsorbent. Analysis in such a case is performed using a specific analytical method developed for the individual metabolite.

Another physical property of the substrate that can change during metabolism is the electrical conductivity. By measuring the change in conductivity after a certain incubation time, the degree of metabolic activity can be determined.

For the better understanding of the invention, reference will be made in the following description to the accompanying drawings, in which:

Figure 1 shows a calibration curve for a particular microorganism, in which the metabolic rate (measured as change in absorbance) is plotted against the number of microorganisms, N;

Figure 2 shows a calibration curve for vitamin C, in which the concentration of the vitamin is plotted against the change in the absorbance of the substrate after a given incubation time with a known number of microorganisms;

Figure 3 shows a calibration curve for a known antibiotic, in which the concentration of the antibiotic is plotted against the absorbance of the substrate; and

Figure 4 shows a simple unit for the practical performance of the present invention.

In the present invention, it is necessary 1) to use a suitable adsorbent to which the microorganisms will bind, 2) to have a washing unit where the adsorbent with bound cells is washed to remove all unspecifically bound material, and 3) to have a unit where adsorbed cells are supplied with nutrients so that metabolism can take place.

The procedure is made quantitative by preparing calibration curves using the results obtained with known amounts of particular microorganisms. The calibration curves are based on changes in chemical or physical parameters that take place as a result of the metabolic activity of the microorganism. These parameters can be acidity (i.e. pH value), oxygen pressure, redox level, electrical conductivity, concentration of a certain metabolite, carbon dioxide pressure, etc.

One of the most useful parameters is the pH of the substrate. To determine this a pH-meter may be used, but in most cases a pH-indicator is more convenient, the colour of which is related to the pH of the solution. Such pH-indicators may be of very different chemical structures. Several different ones have been used in the present invention. Examples of suitable substances are heptametoxired and phenol red. Usually the indicator is added beforehand to the nutrient medium; after incubation the medium is analysed, usually spectrophotometrically. After the three step procedure has been calibrated using known amounts of a particular microorganism, the analytical system can be used to determine the amount of that microorganism in an unknown sample. The adsorption of the microorganism is achieved by using a carrier substance to which are attached antibodies which specifically bind that particular microorganism. The carrier is usually a gel with a low tendency for unspecific adsorption of microorganisms and other biological material. A particularly useful carrier is Sepharose (registered Trade Mark). The carrier must be modified by attaching antibodies to it which have a specificity towards the particular microorganism which is to be measured. In certain cases it may be of interest to collect the whole range of microorganisms in a sample, and an adsorbent with the ability to generally adsorb all microorganisms must then be used. Such an adsorbent can having binding structures of the lectin type, which are known to generally have a much broader substrate specificity than specific antibodies.

In order to measure the amount of a particular microorganism in a mixture of microorganisms, an adsorbent is used which binds specifically to that microorganism. The adsorbent with its binding structures, either lectins or antibodies, is brought into contact with the mixture of microorganisms for a given period of time and then washed to remove unspecifically bound cells.

The washed adsorbent with bound microorganisms is then supplied with nutrient, which can be a sugar solution with suitable buffering substances and, for example, a pH-indicator, or some other liquid substrate in which the bound cells can metabolise.

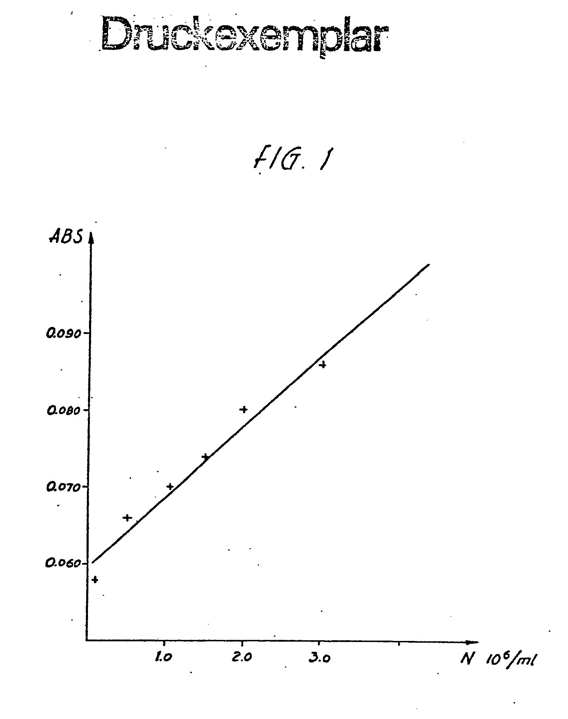

When the adsorbent with bound microorganisms is supplied with the nutrient medium, metabolism begins and is allowed to proceed for a certain number of minutes, usually 30—120. After this incubation period, the nutrient medium is analysed using a suitable parameter, usually absorbance. With the measurement obtained, it is possible, by consulting a calibration curve (Figure 1) previously prepared using various known amounts of ·the particular microorganism, to determine the amount of that microorganism present in the sample mixture. The measurement of absorbance is a convenient way to determine the change in the pH of the substrate resulting from metabolic activity.

In many examples of metabolic activity there is a demand for externally supplied oxygen. The amount of oxygen consumed can be measured and from this value it is possible to determine the number of bound microorganisms using a calibration curve similar to that shown in Figure 1. Similarly, the number of redox equivalents formed or the amount of carbon dioxide generated by the metabolic activity can be measured. The values obtained are directly related to the number of bound microorganisms.

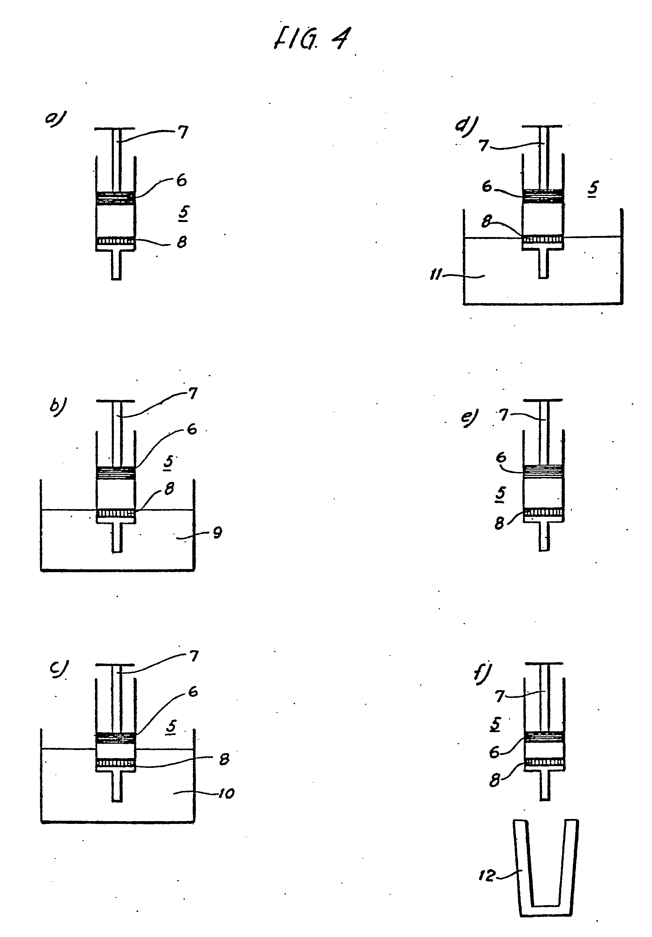

The methods described by which the number of microorganisms in a sample can be determined generally use laboratory equipment familiar to chemists. However, it is possible to use the present invention in a simpler way provided that a calibration curve of the type shown in Figure 1 is available and that it is possible, by the use of colour charts or spectrophotometry, to measure the change in colour that takes place in the nutrient medium as a result of metabolic activity by the bound microorganisms. The method is illustrated in Figure 4 with the subfigures 4A—4F.

These figures show a disposable plastic syringe 5 with a piston 6 and a plunger 7. Inside the syringe and across the outlet is placed a screen 8 made, for example, of nylon mesh with holes of 25 μm. On top of the screen is placed the adsorbent provided with antibodies or other binding structures specific towards the particular microorganisms to be measured.

In Figure 4b, a measured sample 9 of the microorganism solution to be analysed, is drawn up into the syringe where the adsorbent binds the microorganism to which it is specific (the amount of adsorbent used should, of course, be sufficient to bind all the microorganism that is likely to be encountered in the sample). The excess liquid is then expelled from the syringe and the latter, see Figure 4c, is dipped into water or other washing liquid 10 which is drawn up into the syringe to effect washing of the sorbent. Non-bound microorganisms and other impurities are then expelled with the washing liquid from the syringe.

In the next stage, Figure 4d, a sufficient amount of nutrient medium 11 is drawn up into the syringe and mixed with the adsorbent and bound microorganisms. The nutrient medium may consist of a buffered glucose solution with a suitable indicator, or other suitable substances.

The nutrient medium is kept in the syringe and the mixture is allowed to incubate for a certain time, usually between 30 and 120 minutes, sometimes shorter or longer Figure 4e. After this time, the nutrient medium is emptied from the syringe into a vessel 12 which is preferably a transparent cuvette (Figure 4f). By measuring the absorbence of the nutrient medium, the amount of the particular microorganism in the sample 9 can be determined using a suitable calibration curve.

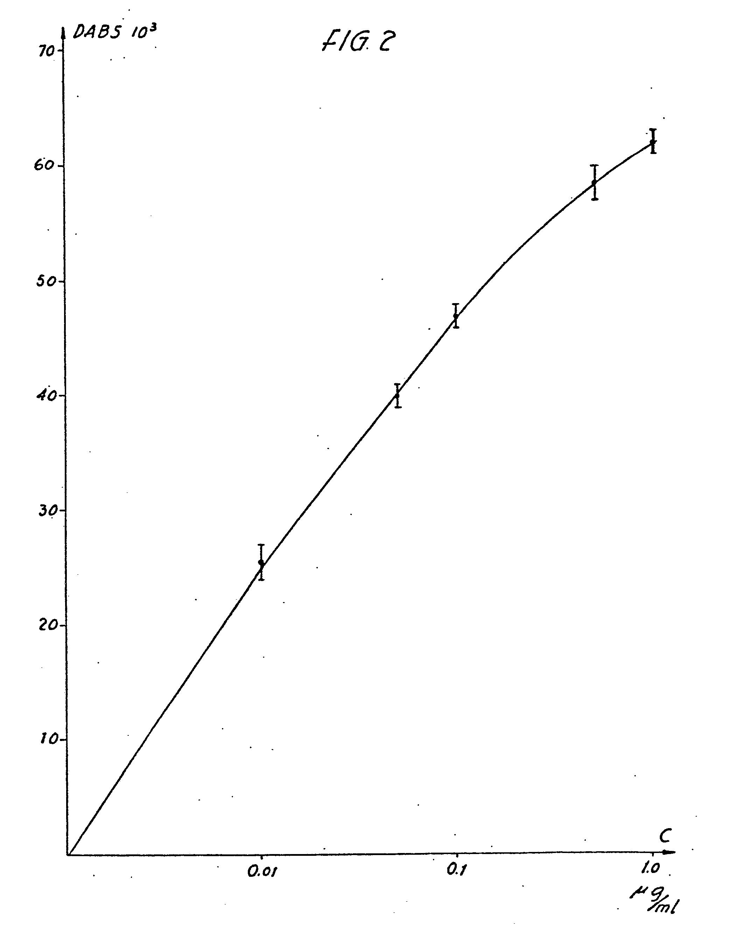

The procedure according to the present invention can also be used to determine the concentration of substances, such as vitamins. The procedure involves the selection of a microorganism that needs an external supply of the substance of interest in order to sustain metabolism. The adsorbent bearing a known amount of such a microorganism is washed in the usual way and then contacted with the substance to be measured. The adsorbent is then washed to a nutrient medium and the resultant metabolic activity is measured after a given incubation time. Again changes in the pH of the substrate can be used to measure metabolic activity and in this way a calibration curve can be prepared using various known amounts of the substance. The curve can then be used to determine the amount of the substrance present in an unknown sample. Figure 2 shows a calibration curve relating to the absorbance of the substrate to the amount of vitamin C present.

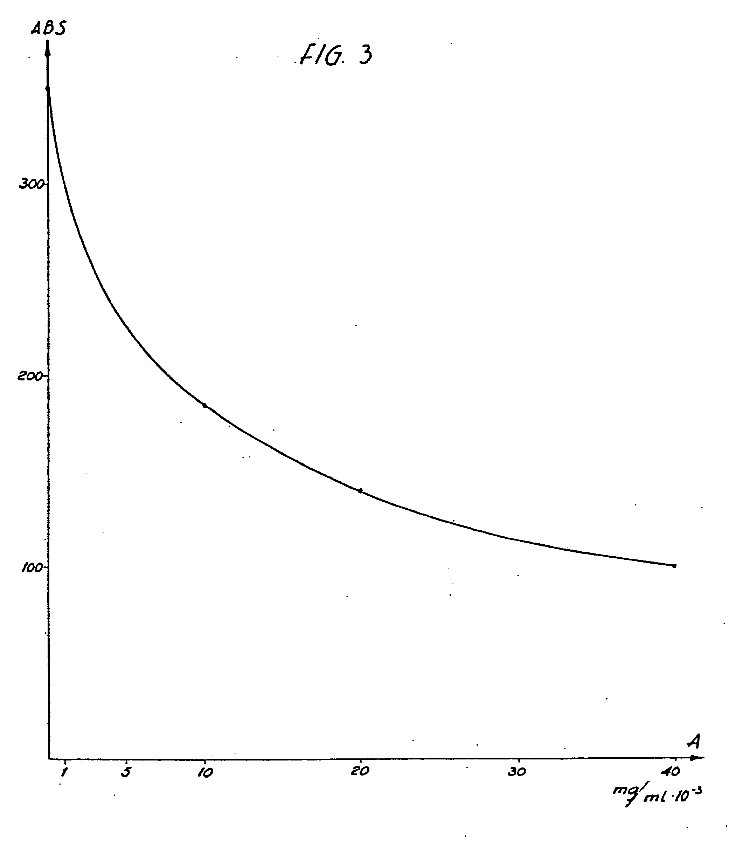

The procedure according to the present invention can also be used to determine the concentration of inhibitory substances such as antibiotics, by studying the effect of the antibiotic on the metabolism of various microorganisms. In this procedure, the adsorbent bound with a known amount of microorganisms is washed in the usual way and then contacted with the antibiotic. The adsorbent is then added to a nutrient medium; metabolism begins, but it is inhibited to a certain extent by the presence of the antibiotic. By measuring the absorbance of the nutrient medium after a given incubation time, usually between 30 and 120 minutes, the inhibitory effect of the antibiotic can be determined. Figure 3 shows a calibration curve obtained using a known number of microorganisms incubated in the presence of various concentrations of the antibiotic. Using such a calibration curve, absorbance values obtained with unknown samples can be used to determine the amount of antibiotic present. This test also provides information about the pattern of resistance of the microorganism.

All cells contain ATP; this fact can be used to determine the number of cells in a sample. The microorganisms are bound to a specific adsorbent in the usual way and the bound cells are then treated in such a way that ATP is released into the substrate surrounding the adsorbent. It has been shown empirically that the amount of ATP released is directly related to the number of cells. Addition of the enzyme-substrate system Puciferase-Puciferin produces a light signal which can be related to the amount of ATP in the sample and thus to the number of microorganisms present in the original sample.

The present invention is also concerned with another important area of general interest, namely the concentration of the microorganisms in a sample into a smaller volume in order to obtain a more sensitive and accurate analysis.

In order that the invention may be more fully understood, the following examples are given by way of illustration only.

Example 1
(Quantitative determination of yeast cells)

A disposable plastic syringe (2 ml) with a screen having 25 μm openings placed across the outlet was used. 1 ml of a suspension of 0.5 ml of sedimented Concanavalin A Sepharose (either prepared by conventional procedures or brought from Pharmacia Fine Chemicals AB, Uppsala, Sweden) in 2 ml buffer, pH 7.4 (0.004 mM $KH_2PO_4$, 0.054 mM Tris, 0.41 mM $NaHCO_3$, 0.95 mM $CaCl_2$, 0.80 mM $MgSO_4$, 5.36 mM KCl, 137 mM NaCl) was poured into the syringe (with the piston and plunger removed). The piston was introduced and the air expelled, followed by the excess liquid. The sample of yeast cells to be analysed was drawn up into the syringe. A total of 2 ml of sample was used. 10 minutes was allowed for the cells to bind to the gel. During binding, the contents of the syringe were mixed gently by turning the syring end-over-end at intervals of 1—3 minutes. The excess liquid is then expelled from the syring and 2 ml of aqueous buffer was drawn in as the wash liquid. After mixing, the wash liquid was expelled. This washing procedure was repeated four times, then 2 ml of a substrate solution for yeast cells was drawn in, after which the syringe was shaken to give an homogenous suspension. The excess substrate was expelled so that 0.8 ml (total volume=substrate+gel) remained in the syringe.

The substrate consisted of buffer of the composition given above containing 50 mM of glucose and 0.2 mg/ml of neutral red. The addition of indicator to the substrate allowed the initial pH to be determined.

Incubation was carried out in a water bath at 26°C for 2 hours. During this time mixing was carried out by occasional shaking of the syringe.

After the incubation period, the substrate solution was expelled from the syringe into a transparent cuvette and the absorbance at 528 nm was measured.

| Number of cells | Change in Abs. 528 |
| --- | --- |
| $4 \times 10^4$ | 0.054 |
| $7 \times 10^4$ | 0.057 |
| $1.1 \times 10^5$ | 0.078 |
| $1.5 \times 10^5$ | 0.085 |
| $2.2 \times 10^5$ | 0.088 |
| $2.5 \times 10^5$ | 0.095 |
| $7.5 \times 10^5$ | 0.107 |
| $1.0 \times 10^5$ | 0.119 |

Example 2
Quantitative determination of yeast cells in suspensions of various concentrations.

In this example, a 2 ml disposable plastic syringe was used, into which 0.5 ml of sedimented Concanavalin-S-Sepharose was introduced according to the procedure described in the previous example. This small column was then used as an adsorbent through which the suspension to be analysed were drawn at a constant rate. Standard suspensions of various concentrations of yeast cells were prepared by diluting a yeast cell suspension of known concentration with the buffer described in Example 1.

The volume of each of the samples drawn through the adsorbent was adjusted with respect to the cell content, so that in each case an equal number of cells was passed through the Concanavaline A-Sepharose column. When each cell suspension had been drawn through the column, binding was allowed to take place for 10 minutes to allow time for the creation of strong multipoint attachment bonds. The experimental procedure then followed that given in Example 1.

In each experiment, except for the blank, $6 \times 10^6$ cells were bound to the adsorbent.

| No. of cells per ml (total no. of cells $6 \times 10^6$) | Abs.$_{528}$ |
| --- | --- |
| 0 | 0.020 |
| $0.1 \times 10^6$ | 0.080 |
| $0.2 \times 10^6$ | 0.116 |
| $0.6 \times 10^6$ | 0.115 |
| $1.2 \times 10^6$ | 0.120 |
| $6 \times 10^6$ | 0.122 |

Example 3
Quantitative determination of thiamin by yeast cells

The practical details were essentially those given in Example 1, but using different buffer, substrate and indicator. Furthermore, the initial condition of the cells was quite different. They were grown on special Difcos medium for thiamine assay. (The cells were grown for two days according to the Difcos manual).

Buffer: 0.1 M sodium acetate/acetic acid, pH 4.5, containing 0.95 mM $CaCl_2$, 0.81 mM $MgSO_4$, 5.36 mM KCl, 137 mM NaCl and 1.0 mM $MnCl_2$.

Substrate: 50 mM glucose and 4 mg/l bromophenol blue indicator in the above buffer.

Thiamin solution: After binding the yeast cells to the Concanavalin A-Sepharose, the syringe was filled (2 ml) with a thiamine solution or a sample solution in which the content of thiamin was to be determined. Absorption of the thiamin was carried out at 37°C for $\frac{1}{2}$ hour with regular mixing. The adsorbent was then washed twice with buffer.

The substrate was then added to the syringe. Incubation was carried out at 37° for 2 hours with gentle mixing. The substrate solution was then removed and the absorbance at 585 nm measured.

| Thiamine (ug/ml) | Abs.$_{585}$ |
| --- | --- |
| 0 | 0 |
| 0.1 | 0.0253 |
| 0.5 | 0.040 |
| 0.10 | 0.047 |
| 0.50 | 0.0585 |
| 1.0 | 0.062 |

Example 4
Quantitative determination of E. coli cells

The basic experimental procedure was the same as that given in Example 1.

Buffer: CaCl$_2$ 0.95 mM, KCl 5.35 mM, NaCl 137 mM, MgSO$_4$ 0.80 mM, KH$_2$PO$_4$ 0.004 mM NaHCO$_3$ 0.04 mM Tris 0.053 mM, pH 7.4.

Substrate 50 mM glucose and 0.2 mg/ml neutral red indicator in the above buffer.

Into a 2 ml disposable syringe was introduced 0.5 ml of sedimented Sepharose 4B provided with antibodies having a binding specificity towards *Hemophilus influencae*, but also a certain activity towards *E. coli*. The coupling technique used is known (C. Borrebaeck, J. Börjesson and B. Mattiasson, *Clin. Chim. Acta, 86,* (1978) 267—278). 20 minutes was allowed for binding to take place. After washing with buffer incubation with substrate was carried out at 37°C for 2 hours. The absorbance of the substrate solution at 528 nm was then measured.

| Number of cells | Abs. $_{528}$ |
| --- | --- |
| $1 \times 10^6$ | 0.030 |
| $2 \times 10^6$ | 0.058 |
| $3 \times 10^6$ | 0.080 |
| $4 \times 10^6$ | 0.103 |
| $5 \times 10^6$ | 0.110 |

Example 5
Qantitative determination of amphotericin

Quantitative determination of amphotericin was carried out by measuring the inhibiting effect of amphotericin on the metabolic activity of bound microorganisms.

The procedure followed that of Example 1, with the exception that 6 ml syringes were used and the adsorbent was contacted with various concentrations of amphotericin for 20 min prior to incubation with the substrate.

| Amphotericin (ug/ml) | Abs.$_{528}$ |
| --- | --- |
| 0 | 0.067 |
| 2 | 0.055 |
| 4 | 0.051 |
| 8 | 0.042 |
| 16 | 0.038 |
| 32 | 0.041 |

**Claims**

1. A method of quantitatively assaying a microorganism or unicellular organism present in a liquid medium, which comprises contacting the medium with an adsorbent in the form of a carrier provided with lectins and/or antibodies, which lectins and/or antibodies bind specifically to the microorganism or unicellular organism to be assayed, separating the adsorbent and the bound organism from any non-bound organisms, contacting the adsorbent and the bound organism with a substrate for the metabolism of the bound organism, measuring a chemical or physical change in the substrate which results from the metabolism of the bound organism, and assaying the amount of the bound organism from such measurement.

2. A method of quantitatively assaying a substance, in which a known amount of a microorganism or unicellular organism, the metabolism of which is affected, positively or negatively, by the substance to be assayed, is bound on an adsorbent as specified in claim 1, the adsorbent and the bound organism are contacted with a liquid medium containing the substance to be assayed, a chemical or physical change in the medium resulting from the metabolism of the bound organism is measured, and the amount of the substance present in the medium is assayed from such measurement.

**Patentansprüche**

1. Verfahren zur quantitativen Bestimmung eines Mikroorganismus oder Einzellerorganismus, der sich in einem flüssigen Medium befindet, *dadurch gekennzeichnet*, daß das Medium mit einem Adsorbens in Form einer Trägersubstanz in Berührung gebracht wird, die mit Lectinen und/oder Antikörpern ausgerüstet ist, welche Lectine und/oder Antikörper spezifisch an den zu bestimmenden Mikroorganismus oder den Einzellerorganismus binden, das Adsorbens und der gebundene Organismus von nichtgebundenen Organismen abgetrennt wird, das Adsorbens und der gebundene Organismus mit einem Substrat für den Metabolismus des gebundenen Organismus in Berührung gebracht wird, eine chemische oder physikalische Änderung in dem Substrat gemessen wird, die sich aus dem Metabolismus des gebundenen Organismus ergibt, und die Menge des gebundenen Organismus aus einer derartigen Messung bestimmt wird.

2. Verfahren zur quantitativen Bestimmung einer Substanz, *dadurch gekennzeichnet,* daß eine bekannte Menge eines Mikroorganismus oder Einzellerorganismus, deren Metabolismus positiv oder negativ durch die zu bestimmende Substanz beeinflußt wird, an ein Adsorbens gemäß Anspruch 1 gebunden wird, das Adsorbens und der gebundene Organismus mit einem flüssigen Medium in Berührung gebracht werden, das die zu bestimmende Substanz enthält, eine chemische oder physikalische Änderung in dem Medium, die sich aus dem Metabolismus des gebundenen Organismus ergibt, gemessen wird, und die Menge der in dem Medium vorhandenen Substanz aus einer derartigen Messung bestimmt wird.

**Revendications**

1. Procédé de détermination quantitative d'un microorganisme ou d'un organisme unicellulaire présent dans un milieu liquide, qui consiste à mettre en contact le milieu avec un adsorbant sous la forme d'un support muni de lectines et/ou d'anticorps, lesquels lectines et/ou anticorps se lient spécifiquement au microorganisme ou organisme unicellulaire à déterminer, à séparer l'adsorbant et l'organisme lié des organismes éventuellement non liés, à mettre en contact l'adsorbant et l'organisme lié avec un substrat pour le métabolisme de l'organisme lié, à mesurer une modification chimique ou physique dans le substrat qui résulte du métabolisme de l'organisme lié, et à déterminer la quantité de l'organisme lié à partir d'une telle mesure.

2. Procédé de détermination quantitative d'une substance, dans lequel une quantité connue d'un microorganisme ou d'un organisme unicellulaire, dont le métabolisme est affecté, positivement ou négativement, par la substance à déterminer, est liée sur un adsorbant comme il est indiqué dans la revendication 1, l'adsorbant et l'organisme lié sont mis en contact avec un milieu liquide contenant la substance à déterminer, une modification chimique ou physique dans le milieu résultant du métabolisme de l'organisme lié et mesurée, et la quantité de substance présente dans le milieu est déterminée à partir de cette mesure.

Druckexemplar

FIG. 1

FIG. 2

FIG. 3

## FIG. 4

a)

b)

c)

d)

e)

f)